(19) Europäisches Patentamt
European Patent Office
Office européen des brevets

(11) **EP 3 943 185 A1**

(12) **EUROPEAN PATENT APPLICATION**
published in accordance with Art. 153(4) EPC

(43) Date of publication:
**26.01.2022 Bulletin 2022/04**

(21) Application number: **20773286.8**

(22) Date of filing: **10.03.2020**

(51) International Patent Classification (IPC):
**B01J 4/02** *(2006.01)*   **B01L 3/02** *(2006.01)*
**G01N 35/10** *(2006.01)*

(52) Cooperative Patent Classification (CPC):
**B01J 4/02; B01L 3/02; G01N 35/10**

(86) International application number:
**PCT/JP2020/010170**

(87) International publication number:
**WO 2020/189395 (24.09.2020 Gazette 2020/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.03.2019  JP 2019051414**

(71) Applicant: **Panasonic Intellectual Property
Management Co., Ltd.
Osaka-shi, Osaka 540-6207 (JP)**

(72) Inventor: **NAKAJIMA, Hisato
Osaka-shi, Osaka 540-6207 (JP)**

(74) Representative: **Eisenführ Speiser
Patentanwälte Rechtsanwälte PartGmbB
Postfach 31 02 60
80102 München (DE)**

(54) **DISPENSING METHOD AND DISPENSING DEVICE**

(57)    This dispensing method includes a connection step for connecting a flange part (64) of a piston (65) and a drive member (50) that drives the piston (65), a first movement step for causing the drive member (50) to move to a first position so as to come into contact with the lower surface of the flange part (64), an intake step for raising the piston (65) to thereby take a liquid into a syringe (62) into which the piston (65) is inserted, a second movement step for causing the drive member (50) to move to a second position so as to come into contact with the upper surface of the flange part (64), a discharge step for lowering the piston (65) to thereby discharge the liquid inside the syringe (62), and a separation step for separating the flange part (64) and the drive member (50).

FIG. 1

EP 3 943 185 A1

## Description

Technical Field

The present disclosure relates to a dispensing method for sucking and injecting various liquids required in, for example, cultivation processes in bio-related technology or manufacturing processes of pharmaceutical products, and a dispensing apparatus for performing the method.

Background Art

As a liquid dispensing apparatus used in, for example, cultivation processes in bio-related technology or manufacturing processes of pharmaceutical products, an apparatus using a motor to operate a plunger of a syringe piston that sucks and injects a liquid is conventionally known (see, for example, Patent Literature (hereinafter, abbreviated as PTL) 1).

FIG. 10 illustrates a conventional liquid dispensing apparatus described in PTL 1.

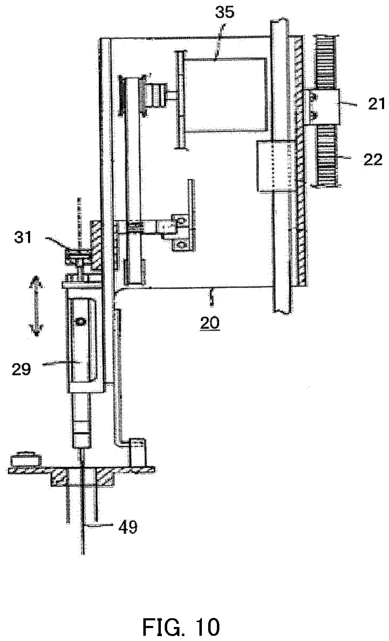

In FIG. 10, syringe 29 moves simultaneously with the movement of syringe carriage 20 on the syringe carriage block, and syringe 29 can be raised and lowered in the upper part of a sample bottle.

Syringe carriage 20 is lowered via traction fitting 21 by driving timing belt 22.

Syringe 29 is lowered together with syringe carriage 20, needle 49 enters a sample, plunger holder 31 is pulled up by plunger drive motor 35, and the sample is drawn into syringe 29 through needle 49, thereby dispensing the liquid.

Citation List

Patent Literature

    PTL 1 Japanese Patent Application Laid-Open No. H7-287003

Summary of Invention

Technical Problem

When the volume of liquid to be dispensed (hereinafter referred to as the "dispensed liquid volume") is large compared to the capacity of syringe 29 (hereinafter referred to as the "syringe capacity"), for example, when 2 ml is to be dispensed with respect to 200 $\mu$l of syringe capacity, it is necessary to repeat the suction and injection operation by syringe 29 many times. On the other hand, when the dispensed liquid volume is small compared to the syringe capacity, for example, when 10 $\mu$l is to be dispensed with respect to 1 ml of syringe capacity, the accuracy of the dispensed liquid volume by syringe 29 generally decreases. It is desirable that a liquid can be dispensed with a small number of suctions and injections and with a small liquid volume error for a wide range of dispensed liquid volumes.

The conventional configuration described in PTL 1 has a problem such that it is not easy to replace a tool (for example, syringe 29) according to a wide range of dispensed liquid volumes. For example, when a tool is replaced manually, there is a possibility of human error, and the time required for the entire dispensing step increases due to the time required to replace the tool and confirm that the replacement has been completed successfully.

The present disclosure is to solve the above-described conventional problems, and an object of the present disclosure is to provide a dispensing method and a dispensing apparatus in which tools can be easily replaced.

Solution to Problem

For achieving the above object, a dispensing method of the present disclosure includes:

    connecting of a flange part of a piston with a drive member that is configured to move the piston;
    first moving of moving the drive member to a first position where the drive member comes into contact with a lower surface of the flange part;
    drawing of a liquid into a syringe by raising the piston, the syringe into which the piston is inserted;
    second moving of moving the drive member to a second position where the drive member comes into contact with an upper surface of the flange part;
    discharging of the liquid contained in the syringe by lowering the piston; and
    separating of the flange part and the drive member from each other.

For achieving the above object, a dispensing apparatus of the present disclosure includes:

    a dispensing mechanism, a piston drive mechanism, and a controller, wherein
    the dispensing mechanism includes a syringe, a piston including a flange part that moves up and down inside the syringe, and a frame to which the syringe is fixed, and
    the piston drive mechanism includes a motor, a drive member that is configured to be driven by the motor and to be in a connection with the flange part to move up and down together with the piston, and a frame to which the motor and the drive member are fixed.

Advantageous Effects of Invention

According to the dispensing method and the dispensing apparatus of the present disclosure, it is possible to easily replace

Brief Description of Drawings

FIG. 1 is a front cross-sectional view of a dispensing apparatus in the present embodiment;
FIG. 2 illustrates the relationship between the dimensions of a flange part and a drive member in the present embodiment;
FIG. 3 is a cross-sectional view of the dispensing apparatus in a first step of the present embodiment;
FIG. 4 is a cross-sectional view of the dispensing apparatus in a second step of the present embodiment;
FIG. 5 is a cross-sectional view of the dispensing apparatus in a third step of the present embodiment;
FIG. 6 is a cross-sectional view of the dispensing apparatus in a fourth step of the present embodiment;
FIG. 7 is a cross-sectional view of the dispensing apparatus in a fifth step of the present embodiment;
FIG. 8 is a cross-sectional view of the dispensing apparatus in a sixth step of the present embodiment;
FIG. 9 is a cross-sectional view of the dispensing apparatus in a seventh step of the present embodiment; and
FIG. 10 illustrates a conventional dispensing apparatus.

Description of Embodiments

Hereinafter, an embodiment of the present disclosure will be described with reference to the drawings. The embodiments shown below are merely examples, and do not exclude the application of various modifications and techniques not specified in the following embodiments. In addition, each configuration of the embodiment can be variously modified and implemented without departing from the scope thereof. Further, each configuration of the embodiment can be selected as needed or can be combined as appropriate.

In all the drawings for explaining the embodiments, the same elements are, in principle, given the same reference numerals, and the description thereof may be omitted.

Configuration of Apparatus

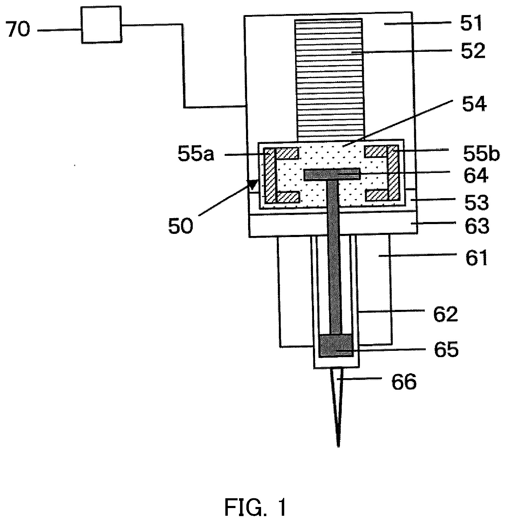

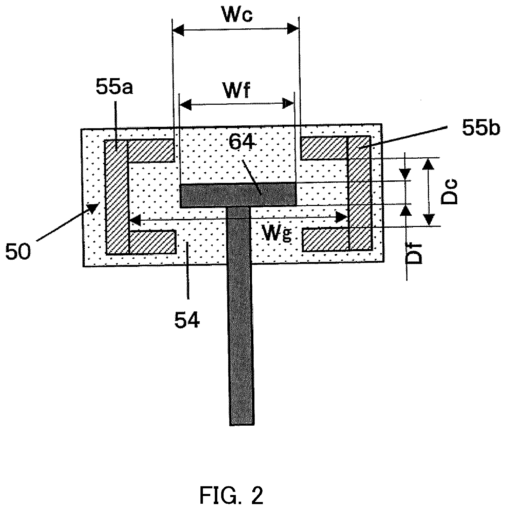

Hereinafter, the configuration of a dispensing apparatus according to the embodiment of the present disclosure will be described in detail with reference to FIGS. 1 and 2. FIG. 1 is a front cross-sectional view of the dispensing apparatus in the present embodiment. FIG. 2 illustrates the relationship between the dimensions of a flange part and a drive member in the present embodiment.

In FIG. 1, master plate 53 of an automatic tool changer system (described below) and motor 52 are attached to frame 51 on the drive member side (hereinafter also referred to as "drive member side frame 51"). Drive member 50 is attached to motor 52, and motor 52 moves drive member 50 up and down.

Drive member 50 includes a pair of left and right chuck parts 55a and 55b, and opening/closing chuck 54 that drives chuck parts 55a and 55b to the left and right.

Opening/closing chuck 54 drives chuck parts 55a and 55b to the left and right by, for example, moving along the guide groove.

Opening/closing chuck 54 drives chuck parts 55a and 55b to the left and right by, for example, pneumatic pressure, but the driving method is not limited to pneumatic, but can be hydraulic, electric, or other driving methods.

Chuck parts 55a and 55b are each driven to the left and right by opening/closing chuck 54, and move synchronously in directions so as to move away from each other or in directions so as to move closer to each other.

Herein, a piston drive mechanism of the present disclosure is configured to include motor 52, drive member 50, and drive member side frame 51.

Syringe 62 for drawing or discharging a liquid is fixed to frame 61 on the syringe side (hereinafter also referred to as "syringe side frame 61"). Further, syringe side frame 61 is provided with tool plate 63 of a tool changer system for connecting syringe side frame 61 and drive member side frame 51.

Piston 65 is movably provided in syringe 62. As piston 65 linked to flange part 64 moves up and down inside syringe 62, liquid is drawn into or discharged out of syringe 62 via tip 66 provided at the end of syringe 62.

Master plate 53 and tool plate 63 form a tool changer system, and linking master plate 53 with tool plate 63 can connect drive member side frame 51 with syringe side frame 61.

The connection part between master plate 53 and tool plate 63 is a mechanism capable of linking master plate 53 and tool plate 63 with each other, and separating master plate 53 and tool plate 63 from each other. As a specific example of such a mechanism, a commonly used mechanism is provided with a claw or a ball on the master plate 53 side and a groove on the tool plate 63 side, and the claw or ball is pneumatically pushed into the groove for linking.

When syringe 62 needs to be changed to match the volume of liquid to be dispensed, tool plate 63 is removed from master plate 53, and then tool plate 63 with syringe 62 having a different capacity fixed thereto is attached to master plate 53.

Herein, a dispensing mechanism of the present disclosure is configured to include syringe 62, piston 65 including flange part 64, and syringe side frame 61.

Controller 70 controls the dispensing apparatus. Controller 70 controls the drive of, for example, motor 52, piston 65, and chuck parts 55a and 55b. That is, controller 70 controls each action of motor 52 and drive member 50. Controller 70 also controls linking and separating of master plate 53 and tool plate 63.

The controller is, for example, the CPU of a computer.

The components included in the dispensing apparatus may be controlled by one controller 70, or may be independently controlled by controllers 70 individually provided for respective components.

FIG. 2 schematically illustrates the relationship between the dimensions of flange part 64 and drive member 50 (chuck parts 55a and 55b, and opening/closing chuck 54) in the present embodiment.

Chuck parts 55a and 55b each have a recess, and are disposed on the left and right such that the recesses face each other.

In the following description, the bottom surface of the recess refers to a surface not facing any surface inside the recess. In other words, the bottom surface of the recess of chuck part 55a refers to the surface whose normal direction is toward the right side of the drawing of FIG. 2, and the bottom surface of the recess of chuck part 55b refers to the surface whose normal direction is toward the left side of the drawing of FIG. 2.

The upper and lower surfaces of the recess are the surfaces respectively disposed at the top and bottom of the recess and facing each other inside the recess. In other words, the upper and lower surfaces of the recess in each of chuck parts 55a and 55b refer to the surfaces whose normal directions are toward the lower side and the upper side of the drawing of FIG. 2, respectively.

Recess height Dc represents the distance between the upper and lower surfaces of the recess.

The material of chuck parts 55a and 55b is, for example, stainless steel, but the material is not limited thereto. The materials of chuck parts 55a and 55b preferably are lightweight, have high rigidity, and are resistant to corrosion from chemicals and the like.

As illustrated in FIG. 2, recess height Dc of each of chuck parts 55a and 55b is larger than thickness Df of flange part 64.

In addition, distance Wc between chuck parts 55a and 55b, namely the distance between the opposing ends of respective chuck parts (the ends are facing each other), is larger than width Wf of flange part 64 when chuck parts 55a and 55b are most open.

Distance Wc between chuck parts 55a and 55b is smaller than width Wf of flange part 64 when chuck parts 55a and 55b are most closed.

Distance Wg between the respective bottom surfaces of the recesses of chuck parts 55a and 55b is larger than width Wf of flange part 64, even when chuck parts 55a and 55b are most closed.

Dispensing Method

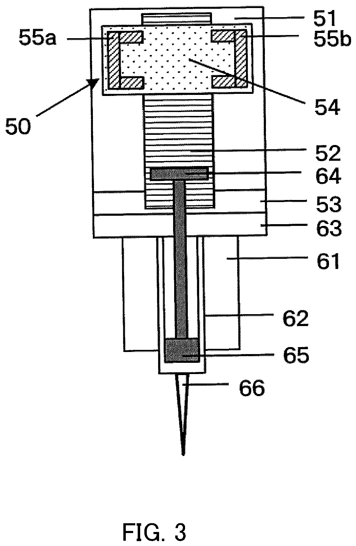

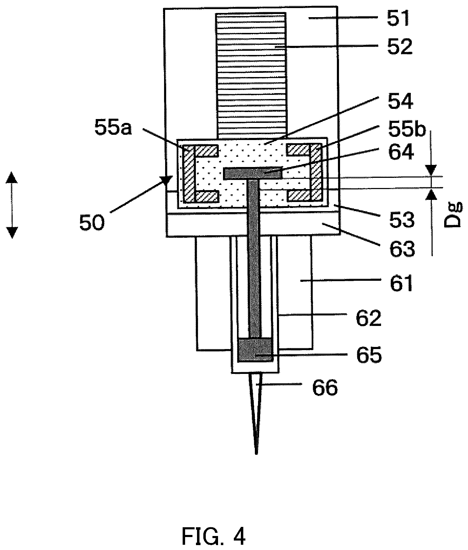

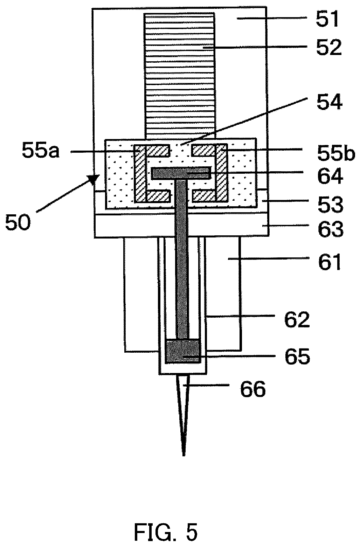

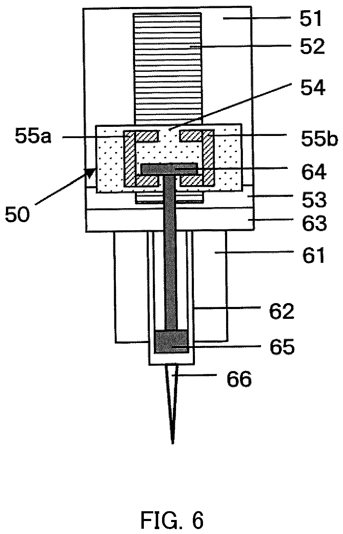

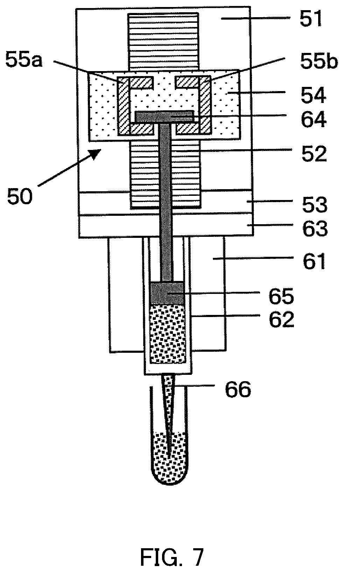

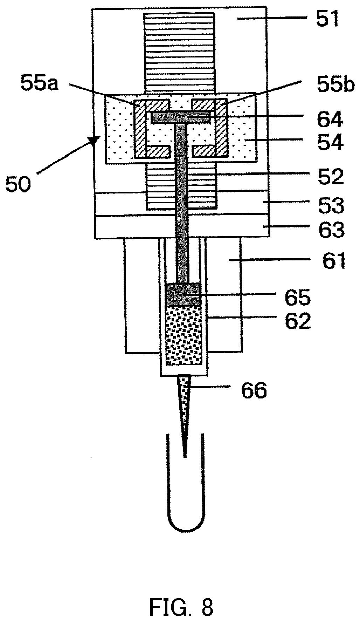

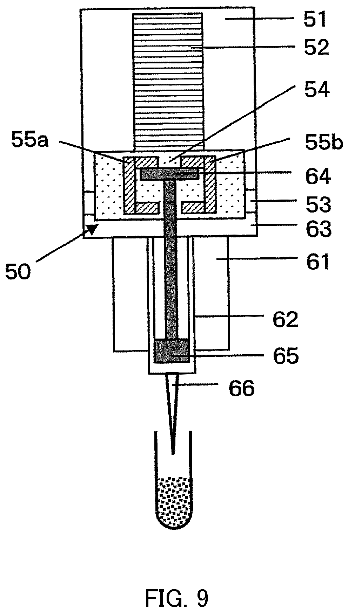

Hereinafter, a dispensing method using the dispensing apparatus of the present disclosure will be described with reference to FIGS. 3 to 9. FIG. 3 is a cross-sectional view of the dispensing apparatus in the first step of the present embodiment. FIG. 4 is a cross-sectional view of the dispensing apparatus in the second step of the present embodiment. FIG. 5 is a cross-sectional view of the dispensing apparatus in the third step of the present embodiment. FIG. 6 is a cross-sectional view of the dispensing apparatus in the fourth step of the present embodiment. FIG. 7 is a cross-sectional view of the dispensing apparatus in the fifth step of the present embodiment. FIG. 8 is a cross-sectional view of the dispensing apparatus in the sixth step of the present embodiment. FIG. 9 is a cross-sectional view of the dispensing apparatus in the seventh step of the present embodiment.

Before drive member side frame 51 and syringe side frame 61 are connected to each other, piston 65 is in a state of being pushed to the tip 66 side in syringe 62 to the maximum extent.

Drive member side frame 51 and syringe side frame 61 are then connected to each other in the first step as illustrated in FIG. 3. In the first step, chuck parts 55a and 55b are in a state of being opened to the maximum. Drive member 50 (chuck parts 55a and 55b and opening/closing chuck 54) is preferably located at the position farthest from syringe 62.

After the first step, motor 52 is activated to lower drive member 50 in the second step illustrated in FIG. 4. The lowering stops when flange part 64 is located between the recesses of chuck parts 55a and 55b, and the space from the lower surfaces of the recesses of chuck parts 55a and 55b (respectively, the inner side surfaces at the lower walls in the recesses of chuck parts 55a and 55b) to the lower surface of flange part 64 has predetermined height Dg. Predetermined height Dg is set so as to satisfy the relationship represented by the expression (1) below from recess height Dc and thickness Df of flange part 64. (See FIG. 2 for recess height Dc and thickness Df of flange part 64.)

$$0 < \mathrm{Dg} < \mathrm{Dc} - \mathrm{Df} \qquad \text{Expression (1)}$$

In the third step illustrated in FIG. 5, chuck parts 55a and 55b are closed most.

The connecting step of the present disclosure is configured to include the first, second, and third steps.

In the fourth step (first moving step) illustrated in FIG. 6 after the above steps, motor 52 is activated to raise drive member 50 to a first position where the lower surface of flange part 64 comes into contact with the inner surfaces at the lower walls (upper surfaces of the lower walls) in the recesses of chuck parts 55a and 55b.

In the fifth step (drawing step) illustrated in FIG. 7, while tip 66 attached to the end of syringe 62 is immersed in a liquid to be dispensed, motor 52 is activated to raise drive member 50 from the first position by a distance corresponding to the dispensed liquid volume to thus raise piston 65, thereby drawing the to be dispensed volume of liquid into syringe 62.

In the sixth step (second moving step) for dispensing the drawn liquid illustrated in FIG. 8, motor 52 is activated to lower drive member 50 to the second position where the upper surface of flange part 64 comes into contact with the inner surfaces at the upper walls in the recesses of chuck parts 55a and 55b. The lowering distance is the difference (= Dc - Df) between recess height Dc of chuck parts 55a and 55b and thickness Df of flange part 64. (See FIG. 2 for recess height Dc and thickness Df of flange part 64.)

In the seventh step (discharging step) illustrated in FIG. 9, motor 52 is activated to lower drive member 50 from the second position by a distance corresponding to the dispensed liquid volume to thus push piston 65 into syringe 62 to the maximum extent, thereby discharging the entire volume of liquid having been drawn into syringe 62.

In the eighth step (separating step) for separating flange part 64 and drive member 50 from each other, the raising is performed from the position where piston 65 is pushed to the maximum extent in syringe 62 as in the seventh step illustrated in FIG. 9 to the position of the third step (connecting step) illustrated in FIG. 5. As a result, a gap is formed between flange part 64 and drive member 50. With the gap formed, chuck parts 55a and 55b are opened, motor 52 is activated to raise drive member 50, thereby completely separating flange part 64 and drive member 50 from each other.

The dispensing method of the present disclosure is achieved by performing the above described first to eighth steps in this order.

For drawing and discharging a liquid, the dispensing apparatus and the dispensing method of the present disclosure allow the liquid to be drawn or discharged with high liquid volume accuracy by eliminating the gap between flange part 64 of piston 65 and drive member 50. For connecting flange part 64 of piston 65 with drive member 50, or separating flange part 64 of piston 65 from drive member 50, by forming the gap between flange part 64 of piston 65 and chuck parts 55a and 55b, flange part 64 of piston 65 can be easily connected with or separated from chuck parts 55a and 55b. Therefore, the tool including syringe 62, piston 65 and the like can be easily replaced according to the volume of liquid to be dispensed.

This application is entitled to and claims the benefit of Japanese Patent Application No. 2019-051414 filed on March 19, 2019, the disclosure of which including the specification, claims, drawings and abstract is incorporated herein by reference in its entirety.

Industrial Applicability

The dispensing method of the present disclosure and the dispensing apparatus performing the method is capable of easy connection or separation of a flange part of a piston with or from a drive member, thereby allowing easy replacement of the piston according to the volume of liquid to be dispensed. Therefore, the dispensing method of the present disclosure and the dispensing apparatus performing the method can be applied to a dispensing process of liquid in cultivation processes in bio-related technology or manufacturing processes of pharmaceutical products.

Reference Signs List

    50 Drive member
    51 Drive member side frame
    52 Motor
    53 Master plate
    54 Opening/closing chuck
    55a Chuck part (right side)
    55b Chuck part (left side)
    61 Syringe side frame
    62 Syringe
    63 Tool plate
    64 Flange part
    65 Piston
    66 Tip
    70 Controller
    Dc Recess height
    Df Thickness of flange part 64
    Dg Height from the lower surfaces of the recesses of chuck parts 55a and 55b to the lower surface of flange part 64 for defining the stop position when drive member 50 is lowered Wc Distance between chuck parts 55a and 55b
    Wf Width of flange part 64
    Wg Distance between respective bottom surfaces of recesses of chuck parts 55a and 55b

**Claims**

1. A dispensing method, comprising:

       connecting a flange part of a piston with a drive member that is configured to move the piston;
       first moving of moving the drive member to a first position where the drive member comes into contact with a lower surface of the flange part;
       drawing a liquid into a syringe by raising the piston, the syringe into which the piston is inserted;
       second moving of moving the drive member to a second position where the drive member comes into contact with an upper surface of the flange part;
       discharging the liquid contained in the syringe by lowering the piston; and
       separating the flange part and the drive member from each other.

**2.** The dispensing method according to claim 1, wherein:

the drive member includes a recess; and
in the connecting, the flange part and the drive member are connected with each other by inserting the flange part into the recess.

**3.** A dispensing apparatus comprising:

a dispensing mechanism, a piston drive mechanism, and a controller, wherein
the dispensing mechanism includes a syringe, a piston including a flange part that moves up and down inside the syringe, and a frame to which the syringe is fixed, and
the piston drive mechanism includes a motor, a drive member that is configured to be driven by the motor and to be in a connection with the flange part to move up and down together with the piston, and a frame to which the motor and the drive member are fixed.

**4.** The dispensing apparatus according to claim 3, wherein:

the drive member includes a recess, and
the drive member is connected with the flange part by inserting the flange part into the recess.

**5.** The dispensing apparatus according to claim 3, wherein:

the drive member includes
a pair of chuck parts each including a recess, the pair of chuck parts being disposed such that the recess of one of the pair of chuck parts faces the recess of the other of the pair of chuck parts.

**6.** The dispensing apparatus according to claim 5, wherein:

the controller controls the pair of chuck parts in such a way that
before the connection with the flange part, a distance between respective ends of the pair of chuck parts is larger than a width of the flange part, and after the connection with the flange part, the distance between the ends is smaller than the width of the flange part, the ends facing each other.

**7.** The dispensing apparatus according to claim 3, wherein:

the controller controls
operations of the motor and the drive member in connecting of the flange part with the drive member,
the operation of the motor in first moving of moving the drive member to a first position where the drive member comes into contact with a lower surface of the flange part, in drawing of a liquid into the syringe, in second moving of moving the drive member to a second position where the drive member comes into contact with an upper surface of the flange part, and in discharging of the liquid contained in the syringe, and
the operations of the motor and the drive member in separating of the flange part and the drive member from each other.

FIG. 1

FIG. 2

FIG. 3

FIG. 4

FIG. 5

FIG. 6

FIG. 7

FIG. 8

FIG. 9

FIG. 10

**INTERNATIONAL SEARCH REPORT**

| International application No. |
|---|
| PCT/JP2020/010170 |

| A. CLASSIFICATION OF SUBJECT MATTER |
|---|
| Int. Cl. B01J4/02(2006.01)i, B01L3/02(2006.01)i, G01N35/10(2006.01)i |
| FI: G01N35/10 D, B01L3/02 D, B01J4/02 B |
| According to International Patent Classification (IPC) or to both national classification and IPC |

| B. FIELDS SEARCHED |
|---|
| Minimum documentation searched (classification system followed by classification symbols) |
| Int. Cl. G01N35/10, G01N1/00, B01J4/00-4/01, B01L3/02, B65D83/76 |

Documentation searched other than minimum documentation to the extent that such documents are included in the fields searched

```
Published examined utility model applications of Japan     1922-1996
Published unexamined utility model applications of Japan   1971-2020
Registered utility model specifications of Japan           1996-2020
Published registered utility model applications of Japan   1994-2020
```

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| A | JP 10-232237 A (SANYO ELECTRIC CO., LTD.) 02 September 1998, paragraphs [0011]-[0018], [0028]-[0036], fig. 1, 2, 4-6 | 1-7 |
| A | JP 59-130528 A (SEIKO INSTRUMENTS INC.) 27 July 1984, page 2, upper left column, line 4 to page 3, upper right column, line 12, fig. 1, 2 | 1-7 |
| A | JP 59-130529 A (SEIKO INSTRUMENTS INC.) 27 July 1984, page 2, upper left column, line 4 to page 3, upper right column, line 20, fig. 1, 2 | 1-7 |
| A | JP 2007-322291 A (SHIMADZU CORP.) 13 December 2007, paragraphs [0037]-[0049], fig. 3, 4 | 1-7 |

☐ Further documents are listed in the continuation of Box C.      ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 20.05.2020 | 02.06.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

INTERNATIONAL SEARCH REPORT
Information on patent family members

| International application No. |
| --- |
| PCT/JP2020/010170 |

| Patent Documents referred to in the Report | Publication Date | Patent Family | Publication Date |
| --- | --- | --- | --- |
| JP 10-232237 A | 02.09.1998 | US 5906795 A fig. 1, 2, 4-6, column 8, line 45 to column 10, line 53, column 12, line 42 to column 14, line 13 EP 801309 A2 JP 9-281115 A JP 9-281114 A JP 9-281117 A JP 10-185930 A JP 10-232235 A JP 10-232236 A JP 10-232238 A | |
| JP 59-130528 A | 27.07.1984 | (Family: none) | |
| JP 59-130529 A | 27.07.1984 | (Family: none) | |
| JP 2007-322291 A | 13.12.2007 | US 2009/0191097 A1 fig. 3, 4, paragraphs [0109]-[0135] WO 2007/139056 A1 CN 101460822 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H7287003 A

- JP 2019051414 A